# EUROPEAN PATENT APPLICATION

(11) **EP 4 339 275 A1**
(43) Date of publication of application: **20.03.2024**
(21) Application number: 22208031.9
(22) Date of filing: 17.11.2022
(51) Int. Cl.: C12M 1/107, C12M 1/42, C12M 1/00, C12P 5/02

(54) **USE OF BIOMAGNETISM FOR BIOGAS PRODUCTION WITH A RECIRCULATION OF FERMENTATION BROTH**

(30) Priority: 15.09.2022 EP 22195898
(71) Applicant: Technische Universität Berlin, 10623 Berlin (DE); Federal University of Pernambuco, 50670-901 Recife (BR)
(72) Inventor: PESSOA, Matheus, 12049 Berlin (DE); KRAUME, Matthias, 16540 Hohen Neuendorf (DE); SOBRINHO, Motta, 50710-485 Recife (BR)
(74) Representative: Hertin und Partner Rechts- und Patentanwälte PartG mbB

(57) **Abstract**

The invention relates to a method for producing biogas. An organic substrate and an enzyme are provided for this purpose. In addition, fermentation broth from a fermenter is recirculated to the enzyme and substrate to form a fermentation broth-enzyme-substrate complex. The fermentation broth-enzyme-substrate complex is exposed to a magnetic field and then returned to the fermenter.

## Description

The invention relates to a method for producing biogas. An organic substrate and an enzyme are provided for this purpose. In addition, fermentation broth from a fermenter is recirculated to the enzyme and substrate to form a fermentation broth-enzyme-substrate complex. The fermentation broth-enzyme-substrate complex is exposed to a magnetic field and then returned to the fermenter.

### Background and state of the art

Biogas is a climate-neutral alternative to natural gas. It is preferably obtained naturally through the decomposition of organic waste or renewable resources and therefore does not count as a fossil fuel. As a renewable source of energy, biogas is mainly compost of three gases: Methane (CH₄), which its chemical energy is converted to mechanical energy, carbon dioxide (CO₂) and in a small proportion but with corrosion potential, hydrogen sulphide (H₂S).

The combustion of biogas can advantageously be CO₂-neutral because plants that are preferably fermented in a biogas plant have absorbed the same amount of CO₂ that is released during the subsequent combustion of biogas. This means that biogas does not cause any additional CO₂ emissions. Moreover, biogas is a good complement to the renewable energy sources, like wind and sun, particularly solar energy. Unlike wind and solar energy, biogas can be produced and stored regardless of the weather. This makes biogas an excellent choice providing baseload supply and balancing fluctuations in the power grid.

For these reasons, there is a high demand for the use and the production of biogas and a great need for continuous improvement. In anaerobic digestion, the production of biogas is directly connected with the level of substrate degradability and its conversion to biogas. To reach a higher production, pretreatments are applied to the substrate (Organic waste, agricultural waste, manure, sludge) aiming at a higher conversion of its carbohydrates, fat and protein content into substrates for the bacteria consortium involved in every step (hydrolysis, acidogenesis, acetogenesis and methanogenesis) for the formation of biogas.

The whole of pretreatments in anaerobic digestion is related to the change in complex substrate structures that weakens carbohydrate-lignin bonds, decreasing its degree of polymerization, increasing particle surface area, particle size reduction, solubility of substrate content, biodegradability enhancement, formation of refractory compounds and loss of organic material. Pretreatments in anaerobic digestion can be classified as thermal, ultrasonic, freeze/thaw, chemical, other mechanical, microwave, wet oxidation, pulsed electric field and biological treatments.

However, the pretreatments described and known from the state of the art, for example by sonification, an increase of enzymatic activity may be achieved, but the energy demand is considerably high.

In the state of the art, approaches are also known to use magnetic fields in the context of biogas production, which can enable a positive effect, particularly as a pretreatment.

For example, in Haritwal et al. (2015), the effect of a magnetic field on the effectiveness of biogas production is investigated. It was found that the increased intensity of the magnetic field led to an increase in the fermentation process. In the studies, cow dung was used, which is an animal waste product, but not an agricultural waste product as such. The effect of the magnetic field takes place during anaerobic fermentation. To provide the magnetic field, a magnetic field device was used, which comprises a transformer core made of cast iron and joined in an E-shape with a DC power supply. The transformer core was mounted on a plastic base and a plastic drum was built. The drum served as a control and was not equipped with a magnetic field.

Zablodskiy, Klendiy and Gritsyuk (2019) investigate the influence of a rotating magnetic field on the intensity of methane formation. In particular, the analysis of physicochemical processes of anaerobic fermentation under the influence of a low-frequency magnetic field is carried out in it in a bioreactor. The results are based on modelling and laboratory studies on the effect of a rotating magnetic field on a water substrate made of animal and plant waste. However, no explicit studies are available in this regard, and discussions regarding enzymes are not made concrete. The enzymes referred to are contained in the fermentation broth.

In Litti et al. (2018), a feasibility study is presented regarding the use of an electromagnetic mill (EM) for mechanical pretreatment of organic waste prior to anaerobic digestion under laboratory conditions. The electromagnetic mill aims to mechanically pretreat and magnetise the substrate. However, the electromagnetic mill consumes a comparatively large amount of energy.

In Zablodskiy et al. (2018), a method for intensifying and controlling the processes of anaerobic fermentation using a magnetic field is disclosed. A rotating alternating magnetic field, a ferromagnetic motor and screw, an electromagnetic field and permanent magnets, and an oscillating field are used. The magnetic field strengths provided are relatively high at approx. 3-3.5 mT.

DE 10 2012 022 178 A1 discloses a process for the production of hydrogen and biogas. In this process, an electrical voltage is provided by means of a permanent magnet. An improvement of methane production to an electrolytic production of hydrogen in the second phase of the fermentation process is attributed to an increase in performance due to electroporation and the electrodynamic process.

In WO 2021/123417 A1, the approach regarding the use of a magnetic field is also followed, but in the context of a pretreatment. In particular, a process for the production of biogas from an organic substrate is disclosed. In this process, an organic substrate is first provided, which is mixed with an enzyme to form an enzyme-substrate complex. The enzyme-substrate complex is subjected to an induced magnetic field as a magnetic pretreatment. This is followed by a temperature treatment of the enzyme-substrate complex exposed to the magnetic field.

The process disclosed in WO 202/12347 A1 already achieves considerable improvements over the known prior art. However, there is a need for optimisation, especially with regard to the quality and quantity of the biogas that can be produced using this process and the transfer to larger-scale bioproduction plants, for example for industrial applications.

### Objective of the invention

The objective of the invention was to improve the biogas and biomethane production of the prior art in terms of efficiency and optimisation. In particular, a successful suitability for highly scalable applications should be made possible.

### Summary of the invention

The problem addressed by the invention is solved by the features of the independent claims. Advantageous embodiments of the invention are described in the dependent claims.

The invention relates preferably to a method to produce biogas from organic substrates comprising:
a. Providing an organic substrate,
b. Providing an enzyme,
c. Recirculating a fermentation broth from a fermenter and mixing the fermentation broth with the organic substrate and the enzyme to form a fermentation broth-enzyme-substrate complex,
d. Exposing the fermentation broth-enzyme-substrate complex to a magnetic field for a time t₁,
e. Introducing magnetically treated fermentation broth-enzyme-substrate complex into the fermenter.

The combination of the preferred steps led to surprisingly beneficial effects that were synergistic. The combination of the preferred steps led to surprisingly beneficial effects that are easily scalable to an industrial biogas plant.

Particularly, it is surprisingly advantageously possible to produce particularly high amounts of biogas through the preferred process. Not only the quantity of the biogas that can be produced is increased, but surprisingly advantageously also the quality.

The surprisingly better quality refers in particular to the higher proportion of methane (which can also be referred as biomethane). The average person is aware that biogas is a gas mixture comprising carbon dioxide and methane. Methane is combustible and can therefore be used to generate energy, for example electricity and/or heat. By means of the preferred process, it is surprisingly advantageous to increase the methane content in the biogas and thus the methane production at low energy consumption.

Furthermore, it is of surprising advantage that this increase of methane is also possible at a higher mass input (high organic loading rate (abbreviated with OLR)), whereby the energy production of a biogas plant can be increased with the common mass input and a higher mass input at the same energy input, since the magnetic field does not require an additional energy input for a higher mass input.

In particular, it is surprisingly advantageously possible to scale up the preferred process to an industrial scale biogas plant. The advantageous scale-up to industrial scale provides an immense usability of the available energy of the producible biogas. Thus, a considerable enrichment in the technical field of energy supply by biogas is achieved by the preferred method.

Furthermore, it is of great advantage that the preferred process, in particular the combination of the preferred steps, can be easily carried out by a skilled person. Therefore, advantageously, in addition to a simple executability, an advantageously high process suitability is given to produce biogas.

Another very great advantage of the preferred process is that only low-cost components and maintenance are needed to carry out the process steps, which is advantageous for the process efficiency as such. To perform pretreatment in anaerobic digestion (biogas), it is necessary to use energy to perform the pretreatment (physical pretreatment). The pretreatment carried out in the preferred process has a low energy requirement, which makes the process efficient in a surprisingly advantageous way.

It should be explained that by means of the preferred process the methane production can be advantageously increased at a high mass input (organic loading rate OLR), as mentioned above. There is a significant advantage by the preferred process in increasing the methane content and production at low energy input. Furthermore, this increase in methane is also advantageously possible with a higher mass input (high organic loading rate), whereby the energy production of the biogas plant can be increased with the common mass input and a higher mass input with the same energy, since the magnetic field used advantageously does not require any additional energy input for a higher mass input.

In particular, the preferred method represents a substantial turning away from the teaching disclosed in WO 2021/123417 A1. In contrast to merely subjecting an enzyme-substrate complex to a magnetic field as a magnetic pretreatment, now preferably a fermentation broth from a fermenter is recycled to the substrate and the enzyme to obtain a fermentation broth-enzyme-substrate complex for a pretreatment. The obtained fermentation broth-enzyme-substrate complex is then exposed to a magnetic field. Thereafter, the magnetically treated fermentation broth-enzyme-substrate complex is preferably introduced into the fermenter.

In particular, the step of recirculating the fermentation broth from the fermenter to combine it with the substrate and enzyme, together with the combination of exposing it to a magnetic field and then introducing it into the fermenter, makes a significant contribution to the overall success of the invention. Advantageously, the preferred method makes it possible to implement the process in a (large-scale) biogas plant. Furthermore, it is of extraordinarily surprising advantage that the fermentation broth from the fermenter preferably has components, such as nutrients, enzymes and/or microorganisms, which have a magnetic sensitivity. Advantageously, the components of the fermentation broth having magnetic sensitivity help to significantly improve the efficiency of the process.

In a further preferred embodiment, an aerobe digestion is initiated. For example, in a biogas plant the preferred anaerobe digestion is initiated already, so that the preferred process can be executed.

By the term magnetic sensitivity, it is meant that if exposed to a magnetic field, they will respond to it. How they respond to the magnetic field depends, among other things, on the type and nature of the constituent. For example, enzymes are known to be three-dimensional structures that can undergo conformational changes (changes in structure) when exposed to a magnetic field. Mention should also be made here of the radical pair mechanism, which may be relevant to the context of the invention, in particular to the theory, but without being limited thereto. The radical pair mechanism preferably provides (among other things) a principle of how magnetic fields can influence chemical reactions. Chemical reactions can be influenced by a magnetic field as a result of four steps, which comprises a physical step involving excitation and/or ionization of the molecules. A physical-chemical stage where charges or neutral radicals are formed. A chemical step where chemical products are formed. The fourth step addresses the biological step been resulted of the previous steps (Bentsman et al., 2006).

Preferably, the term "organic substrate" is known for the person skilled in the art. Preferably, the organic substrate refers to the raw material used in a biogas plant to produce biogas. Occasionally, the term "fermentation substrate" is also used in the prior art. Biomass with a high-water content that cannot be directly thermally utilized is particularly suitable as a substrate. Biomass rich in cellulose (e.g., straw) and lignocellulose (wood) are poorly accessible for microbial degradation and therefore not suitable as a substrate for biogas production without prior degradation of the cellulose by bioextrusion. In bioextrusion, a substrate is preferably squeezed, pressed and crushed by the action of mechanical energy under high pressure and temperature. The mass to be processed is then abruptly relaxed, resulting in the rupture of the cell structure, a lignin phase.

In biogas plants, preferably anaerobic microbial degradation (fermentation) of the organic substrate takes place. The organic substrate serves as a source of nutrients and energy for the microorganisms. The gaseous methane separates from the substrate and can be used, for example, in a combined heat and power plant to generate electricity and heat, as mentioned earlier. The main components of biogas, methane (CH₄) and carbon dioxide (CO₂), are on the one hand metabolic waste of the microorganisms, on the other hand the energy-rich methane is the main product of a biogas plant.

The term enzyme is also known to the person skilled in the art and preferably refers to a substance consisting of large biological molecules that can accelerate certain chemical reactions as a catalyst. In principle, the catalysed reaction can also take place without the respective enzyme, but more slowly and it might be unstable. Most enzymes are proteins; they are formed in the cell like most other proteins via protein biosynthesis at the ribosomes, including the nonribosomal peptide synthetases.

In the sense of the invention, fermentation broth preferably refers to the mixture or parts of the mixture which is introduced and/or present in the fermenter. Fermentation broth preferably means a complex mixture containing organisms, soluble molecules, electrolytes and/or other substances. The components of the fermentation broth may be complex and include microbial cells, colloids, viruses, proteins, polysaccharides, enzymes, individual sugars, organic acids and/or inorganic ions. Fermentation broth advantageously has a higher biogas potential than digestate. Digestate, on the other hand, is preferably fermentation broth that leaves the biogas production process and can be used in particular as a fertiliser, although it also has no or only a very low biogas potential as a result.

Fermenter preferably refers to a device with which a fermentation can be carried out. A fermentation preferably comprises a degradation and/or conversion of organic substances by microorganisms and/or enzymatic-chemical changes by (isolated) enzymes to form certain organic products. Structurally, a fermenter resembles or is similar to a container or a tank.

Preferably, a fermenter is a component of a biogas plant. A biogas plant preferably serves to produce biogas by fermentation of biomass. In particular, the anaerobic (without oxygen) microbial degradation (fermentation) of the (organic) substrate used preferably takes place in a biogas plant.

The fermentation broth-enzyme-substrate complex comprises a mixture comprising fermentation broth, enzyme and substrate. Preferably, fermentation broth is recirculated from the fermenter to combine with the enzyme and substrate to provide the fermentation broth-enzyme substrate complex. A mixture comprising the enzyme and the substrate can also be referred as an enzyme-substrate complex. Preferably, the fermentation broth is taken from the fermenter as a component of the fermentation broth-enzyme-substrate complex. Preferably, the fermentation broth-enzyme substrate complex is magnetically treated under the provision of a magnetic field. Preferably, the magnetically treated fermentation broth-enzyme substrate complex is introduced into the fermenter.

In a further preferred embodiment, the method is characterized in that at least a fraction of the fermentation broth or a whole fermentation broth from the fermenter is recirculated and mixed with the organic substrate and the enzyme to form the fermentation broth-enzyme-substrate complex.

The amount of fermentation broth to be added is preferably the minimum required to make the mixture flowable and/or pumpable.

In preferred embodiments, the ratio of fermentation broth added to the organic substrate and enzyme is between 1 m³ to 1-10 T (ton). Preferably the ratio is 1 to 10, more preferably 1 to 4, most preferably 1 to 1.

Here, the ratio preferably means the ratio (in m³) of the volume of fermentation broth to the mass (in ton) of organic substrate.

Surprisingly, the aforementioned ratios have proven to be advantageous in order to provide a flowable mixture, in particular for the fermentation broth-enzyme substrate complex, on the one hand, and to ensure an extremely successful producibility of biogas, on the other hand.

Furthermore, the aforementioned ratios on the recirculated amount of fermentation broth are surprisingly advantageous in that the producible amount of biogas and/or proportions of the components of the biogas can be adjusted. Since, without being limited to theory, fermentation broth has magnetically sensitive components and the fermentation broth is recirculated to form the fermentation broth-enzyme-substrate complex, which is magnetically treated, the magnetically sensitive components of the fermentation broth in particular have a surprisingly advantageous influence on the production of the biogas.

In a further preferred embodiment, the method is characterized in that the fermentation broth is mixed with the organic substrate and the enzyme in a temperature range between 30 - 70°C, preferred between 35 - 60°C, particularly preferred between 44 - 56°C, very particularly preferred between 40 - 55°C 37 to 55

The specified temperature ranges have surprisingly proven to be particularly advantageous for the formation of the fermentation broth-enzyme-substrate complex, which can be exposed to a magnetic field. The provision of the specified temperature is preferably effected by the recirculation of the fermentation broth, in particular thus by the fermentation broth itself. This is particularly because fermentation broth is preferably recirculated from the fermenter by already having the specified temperatures and/or temperature ranges.

It was completely surprising that an efficient reaction was feasible at the indicated temperature ranges. In particular, the teaching of WO 2021/123417 A1 discloses that a mixture of an organic substrate with an enzyme as well as the exposure of an enzyme-substrate complex should be carried out at room temperature. The present preferred temperature specifications differ significantly from those of room temperature, so that a technically useful completely new and surprising finding has been obtained. Advantageously, it is no longer necessary to provide certain apparatus in order to obtain a desired temperature. Instead, the temperature that can be provided by the fermentation broth (since it comes from the fermenter) can be used. Advantageously, this further improves the process efficiency.

In preferred embodiments, the temperature is above 37°C, but below 70°C, as denaturation of enzymes can occur at or above 70°C. In further preferred embodiments, the temperature is between 35 - 70°C.

In a further preferred embodiment, the method is characterized in that the time t₁ is selected from a range between 1 - 6 h, preferred between 3 - 5 h, particularly preferred 4 h.

It was surprising that a magnetic treatment in the sense of a preferred pretreatment of the fermentation broth-enzyme-substrate complex could advantageously take place in the times mentioned in order to be able to produce a biogas of improved quality, in particular in the form of a higher methane content. Furthermore, advantageously, said times can be easily implemented by a person skilled in the art. In the context of the invention, the time t₁ may also be referred to as the magnetisation time. Furthermore, in the sense of the invention, the exposure of the fermentation broth-enzyme-substrate complex to a magnetic field can preferably be referred as a pretreatment or magnetic pretreatment.

In further preferred embodiments, it is possible to perform an intermittent pretreatment. In the sense of the invention, an intermittent pretreatment preferably means that when pretreatment has already taken place, periods of time can be allowed to elapse without pretreatment taking place. Thereafter, it may be preferred, for example after a few days, like 10 days considering a daily feed, that a pretreatment in the sense of a magnetisation of the fermentation broth-enzyme-substrate complex takes place again. In particular, it may be preferred to make a cycle thereof. Therefore, in a further preferred embodiment, the method is characterised in that the magnetic pretreatment takes place intermittently.

In a further preferred embodiment, the method is characterized in that while forming the fermentation broth-enzyme-substrate complex the fermentation broth-enzyme-substrate is surrounded by a static magnet field.

A static magnetic field has proven to be particularly advantageous for the context of the invention, in particular for the goal of improved biogas producibility. The inventors have recognised that particularly a static magnetic field is excellently suited to magnetically excite the magnetically sensitive components of the fermentation broth-enzyme-substrate complex and to cause such a change and/or reaction that is advantageous for the producibility of biogas. There are several different teachings in the prior art, however the realisation of the use of a static magnetic field provided a surprising advantageous merit to the invention. In particular, the provision and use of a static magnetic field for magnetic pretreatment represents in a sense a choice, especially because there were several, sometimes very different, views in the prior art in this regard.

Furthermore, it is preferred that the magnetic field is essentially homogeneous, i.e., essentially a uniform distribution of the magnetic field strength emanates from the magnetic field. Advantageously, this results in a substantially uniform (or fairly uniform) magnetic activation of the magnetically sensitive components of the fermentation broth-enzyme-substrate complex, which has a beneficial effect on the processing.

In a further preferred embodiment, the method is characterized in that recirculating the fermentation broth from a fermenter is performed by a pump, preferably a positive displacement pump.

Components such as a pump have thus proven to be particularly advantageous for recirculating the fermentation broth from the fermenter in order to mix it with the enzyme and the organic substrate. In particular, it is advantageous to be able to convey the fermentation broth over long distances and/or differences in altitude without any problems. The latter is particularly advantageous in industrial biogas plants.

The aforementioned advantages were surprisingly great when using a positive displacement pump. In the sense of the invention, a positive displacement pump preferably denotes a pump which supplies additional energy to the fermentation broth by means of a mechanical component, for example by means of a piston and/or plunger. In particular, a very fast recirculation of the fermentation broth starting from the fermenter can advantageously take place.

In a further preferred embodiment, the method is characterized in that the magnetic field has a magnetic flux density between 0.01 - 10 mT, preferably 0.2 mT.

It was surprising that an optimal magnetic pretreatment of the fermentation broth-enzyme substrate could be carried out in a complex manner in the specified parameter ranges of the magnetic field, which in turn was surprisingly advantageous for the improved biogas production. In particular, the magnetic sensitive components of the fermentation broth can be magnetically excited surprisingly advantageously at the specified magnetic field strengths.

In a further preferred embodiment, the method is characterized in that the magnetic field is provided by a solenoid through which a DC current flows.

It was surprising that such a magnetic field could be provided using a solenoid fed with DC current, which was particularly advantageous for the magnetic excitation of the fermentation broth-enzyme-substrate complex. In particular, a substantially homogeneous and static magnetic field could be provided with the aid of the solenoid through which direct current flows, which is very useful for magnetic excitation by inducing a static magnetic field. The material for the solenoid can be copper, for example, without being limited to this one.

Furthermore, the provision of the magnetic field by a solenoid with direct current (DC) flowing through it is particularly advantageous for the process efficiency, since a solenoid and a direct current flowing through it are particularly easy to implement. Furthermore, the strength of the magnetic field can advantageously be easily regulated, in particular via the strength of the current fed into the solenoid.

The solenoid is preferably present as a wire. The wire can preferably comprise cooper. The wire is preferably placed in form of a coil. Particularly, in the case of the pretreatment, the solenoid was placed or constructed by surrounding the wire around a pipe were the fermentation broth-enzyme-substrate passes through. In a further preferable embodiment, a pipe or a series of pipes are surrounded by the wire (solenoid). The series of pipes can be place inside a container.

In a further preferred embodiment, the method is characterized in that the organic substrate is selected from a group comprising sugar beet pulp, corn silage, straw and/or gluten, preferred sugar beet pulp.

The organic substrate used according to the invention is preferably sugar beet pulp, which is an agricultural waste from sugar production. However, the process can also be applied to other organic substrates such as corn, corn straw, rapeseed, grain, waste from soy production, rice straw, waste from sugar cane production, etc. In principle, the process can be applied to any organic matter. But for application to other substrates, the same or different enzymes are used and for each different enzyme, the configuration of magnetic field parameters (intensity and/or duration) can change.

Basically, when using the preferred method according to the invention, the enzyme to be used in the process is preferably determined by examining the substrate composition. In the case of sugar beet pulp, pectinase is preferably chosen because of the large amounts of pectin in its composition.

In a further preferred embodiment, the method is characterized in that the enzyme is selected from a group comprising pectinase, cellulase, xylanase and/or glucanase.

The enzymes may be provided in the form of powder or liquid, for example in the form of a solution. Particularly, one enzyme may be used or several enzymes may be combined. In particular, enzyme mixtures and/or enzyme preparations can also be used. The choice of the enzyme also depends on the specific choice of the organic substrate. As indicated above, pectinase is preferred for sugar beet pulp due to the high amount of pectin. Suitable combinations of enzyme and substrate can be made by a person practising the process.

The steps mentioned have proven to be particularly advantageous in the context of the invention to carry out a particularly efficient sonication. In particular, this has proven to be advantageous for the organic substrate, especially against the background of increasing the reaction rate and/or the reaction speed.

In the following, the invention is explained in more detail with reference to figures, without being limited to them.

### Figures

### Brief description of the figures

| **Tab. 1** | Nomenclature in the figures |
|---|---|
| **Fig. 1** | Results of hydrolysis experiments |
| **Fig. 2** | Experimental results regarding a 10 L fermenter in a batch setup |
| **Fig. 3** | Experimental results regarding a 10 L fermenter in a semi-continuous setup |
| **Fig. 4** | Experimental results regarding a 200 L fermenter in semi-continuous setup |
| **Fig. 5** | Comparison of results between the 200 L fermenter and 10 L fermenter |
| **Fig.6** | Results for the corn silage investigations |

### Detailed description of the figures

Tab. 1 shows the meaning of the abbreviations used in the figures below to present experimental results within the context of the invention.

The enzymatic hydrolysis and biogas production (Batch experiments) were carried out with sugar beet pulp pellets (92 % TS) collected from Nordzucker AG.

The semi-continuous operation is been carried out with sugar beet pulp (20-25 % TS) from the biogas plant of Anklam.

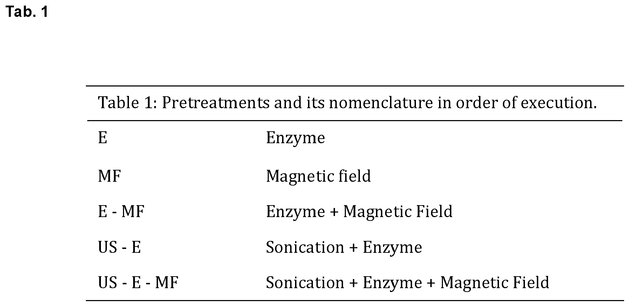

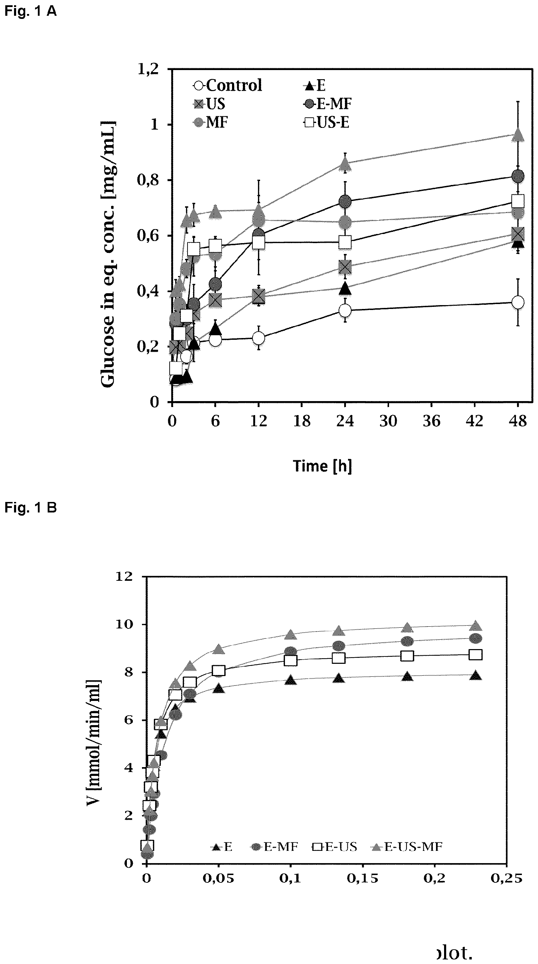

### Goal of Fig. 1: Analysis the release of soluble sugars according to different pretreatments application

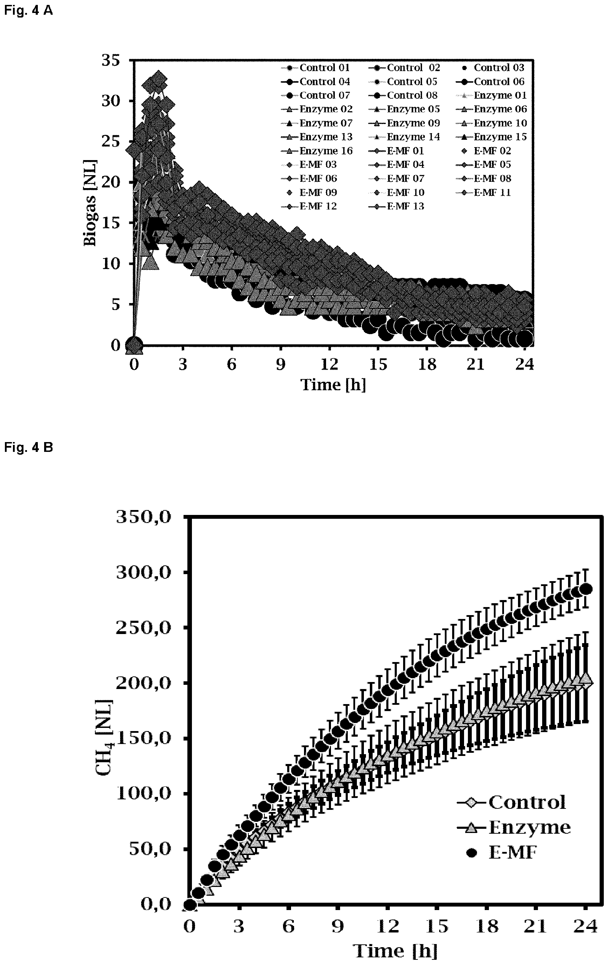

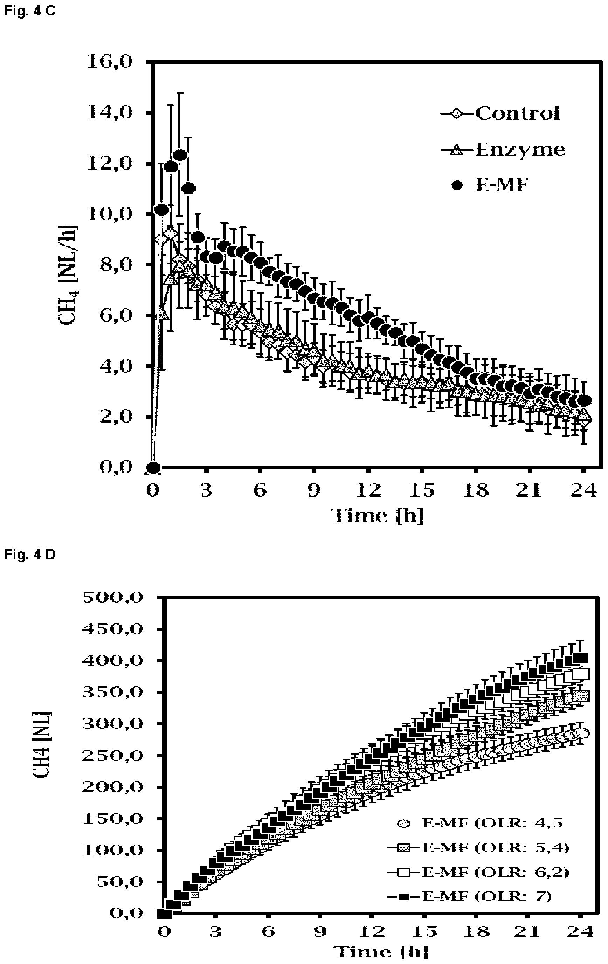

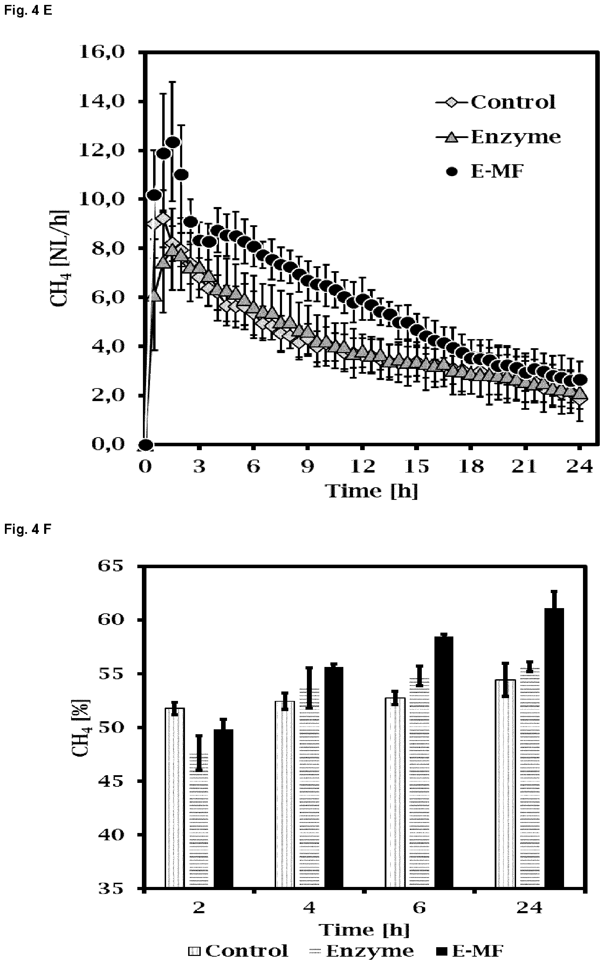

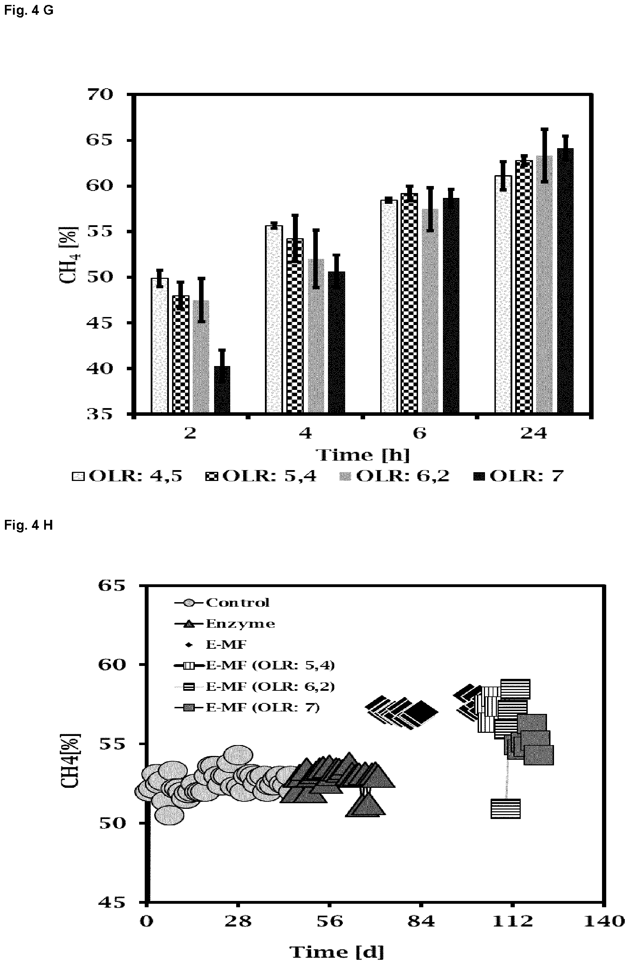

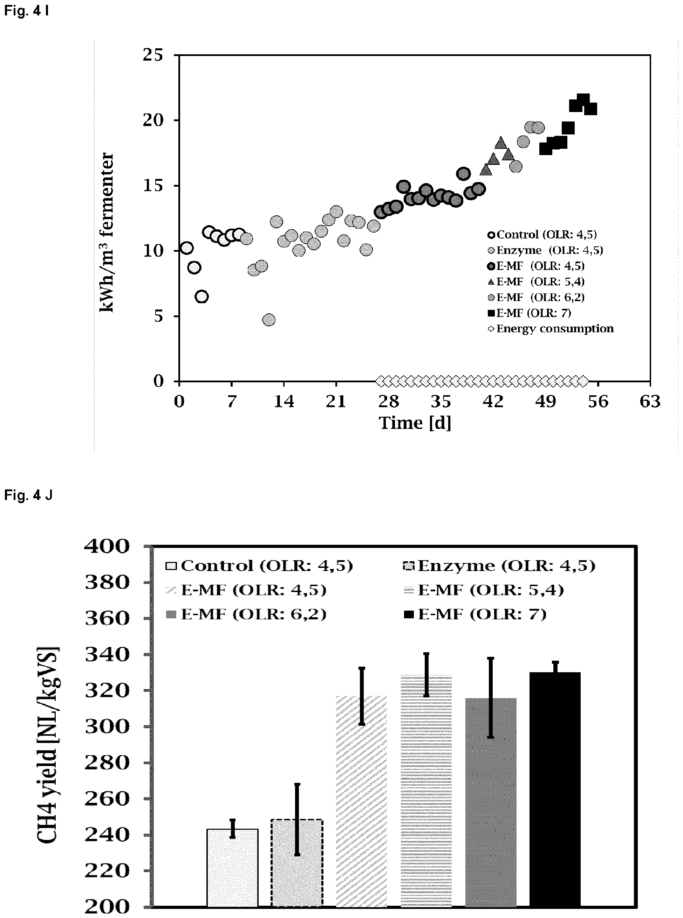

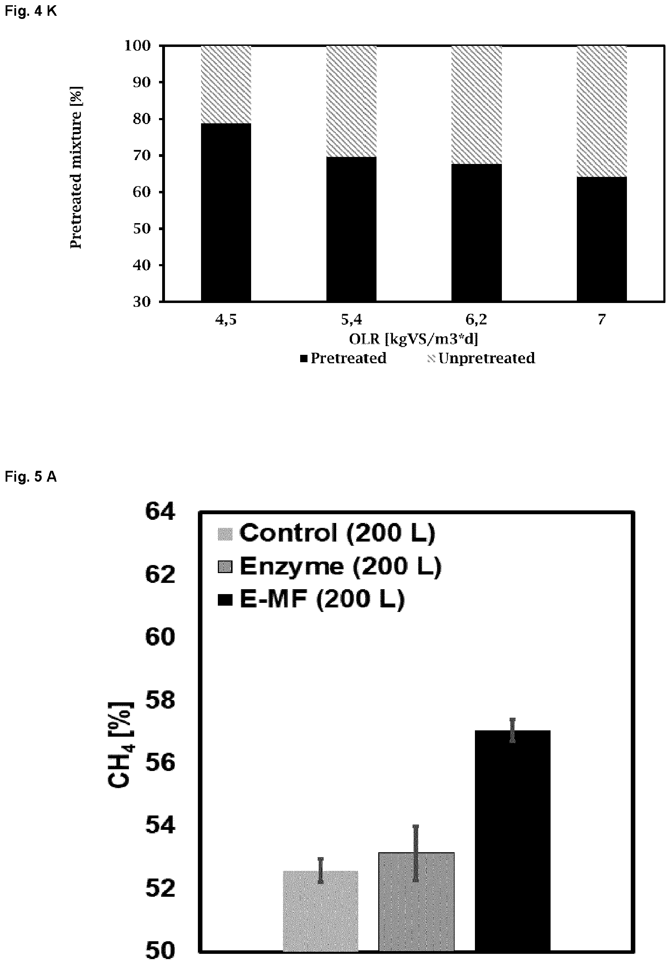

Fig. 1 A shows spectrophotometer analyses of the pretreated samples. It can be seen that as the more pretreatment is applied to the substrate, the more products are dissolved into the liquid phase. The release of soluble sugars according to the applied pretreatment (Figure 4) confirms this trend. A magnetized enzyme substrate complex (E - MF) increased the degradation by 93 % compared to the samples were no pretreatment was applied and 30 % compared to the enzyme alone (E). The combination of three pretreatments presented the highest degradation, 174 % compared to the samples were no pretreatment was applied. The sonication pretreatment also increased the release of soluble sugars in comparison to the control, what was also observed by other authors (Wood et al. (1997), Li et al. (2005)).

The release of soluble sugars for each pretreatment was done by spectrophotometer analysis with a wavelength of 540 nm, pH 4.0 and at 50°C.

### Goal of Fig 1B: Analysis of the increase in enzymatic activity as a result of the application of magnetic field and sonication

**Fig. 1** **B shows a Michaelis-Menten plot.** As it can be seen, sonication, magnetic field and the combination of both led to a higher enzymatic activity within the same hydrolysis time compared to the control, in this case represented by the enzymatic pretreatment. The combination of the ultrasound pretreatment with the enzymatic and magnetic treatment (US-E-MF) reached the highest reaction rate (22 % higher than the enzymatic pretreatment) followed by E-MF (17 %) and E-US (8 %). The different values for Kₘ indicate different Michaelis-Menten constants, i.e., the reactions presented different reactions rate although they had the same substrate concentration.

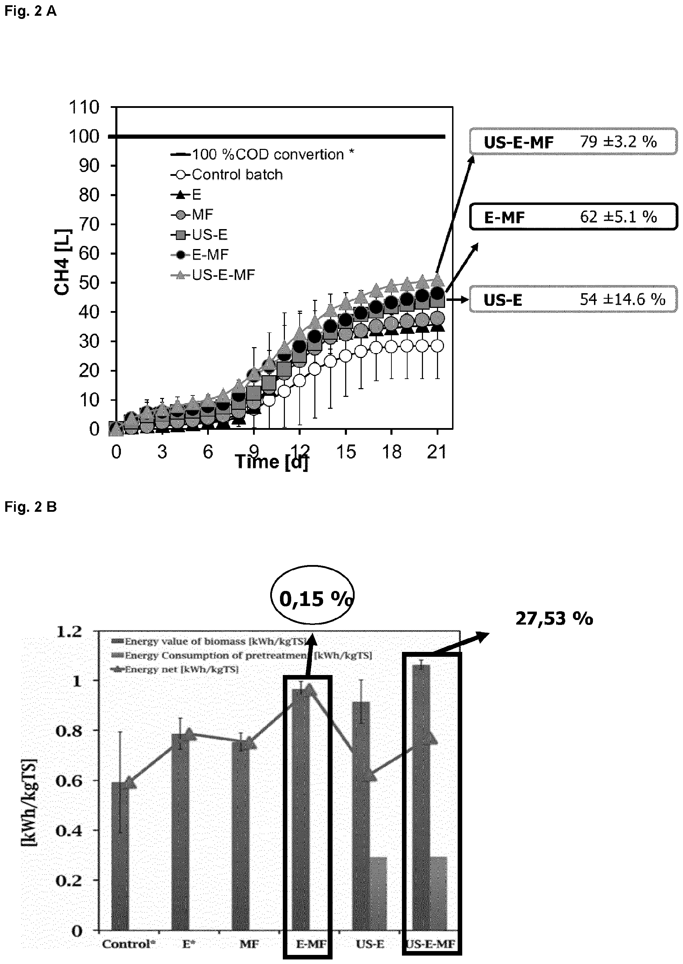

**Fig. 2** **A gives results of methane production in L/d (Liters per day) in the batch mode of the experimental setup.**

**The goal of this set of experiments was to check the influence of the magnetic field on the enzyme-substrate mixture.** For this task, fermentation experiments using only enzymatic pretreatment, and only magnetic field pretreatment were carried out before the fermentation of the magnetization of the enzyme-substrate mixture before fermentation. Also, the influence of sonication to the process was tested. After the application of the pretreatment, or combination of pretreatments, the substrate was led to incubation at 50 °C for the activation of the enzyme prior to fermentation. The biogas and methane production increased after the application of the pretreatments following a reasonable trend. Biogas production presented a clear tendency in the batch tests. The biogas production increases as more pretreatments are applied. The biogas volume increased by 26 ± 4.4 %, for the combination of three pretreatments (US-E-MF) in comparison to the control batch. Data analysis indicates that the pretreatments E-MF, US-E and US-E-MF presented statistical differences in comparison with the control, which was not the case for E and MF when applied alone.

**Fig. 2** **B shows a specific energy gain for the batch experiments.** Error bars were calculated from the duplicate of various pretreatments and the control batch, wherein no energy consumption was accounted for the pretreatment.

After establishing that the application of the E-MF increases the methane percentage in 5 %, the goal of the research aimed to quantify the highest amount of mass per day (Organic loading rate, OLR) that the pretreatment would be able to digest.

For this task, the OLR was stepwise increased until the limit was reached (usually, an industrial biogas plant uses an OLR varying from 2 to 4 kgVS/m³ d). The biogas plant organic substrate is collected from, operates with an OLR of 4,5 as sugar beet pulp is not complicated to digest. This value is considered as the limit in which a biogas plant operates. Operating a biogas plant at higher OLRs than it usually operates will create an instable operation, decreasing the biogas and/or methane production and its methane content. Going to a higher OLR may be possible if the plant increases its temperature in the fermenter to a thermophilic range (50 to 60 °C) but it is not sure if an increase will be achieved at industrial scale. In this experiment the limit of the mesophilic range (44 °C) was stayed and a digestion of higher OLR is possible because the application of the pretreatment provides a more stable anaerobic digestion even at the mesophilic range. The operation of the 10 L fermenter increases the OLR to 11,5, which is almost 3 times more than the common practice.

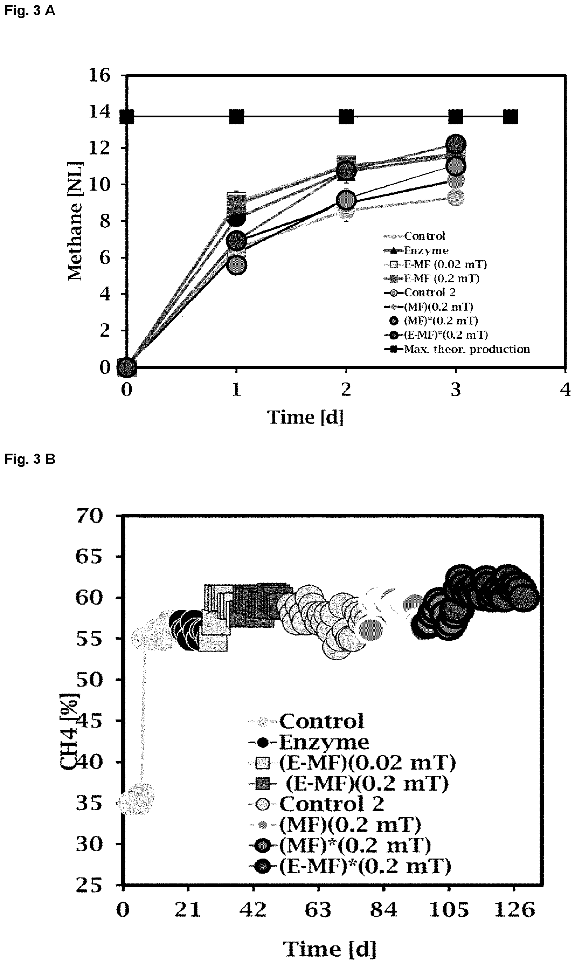

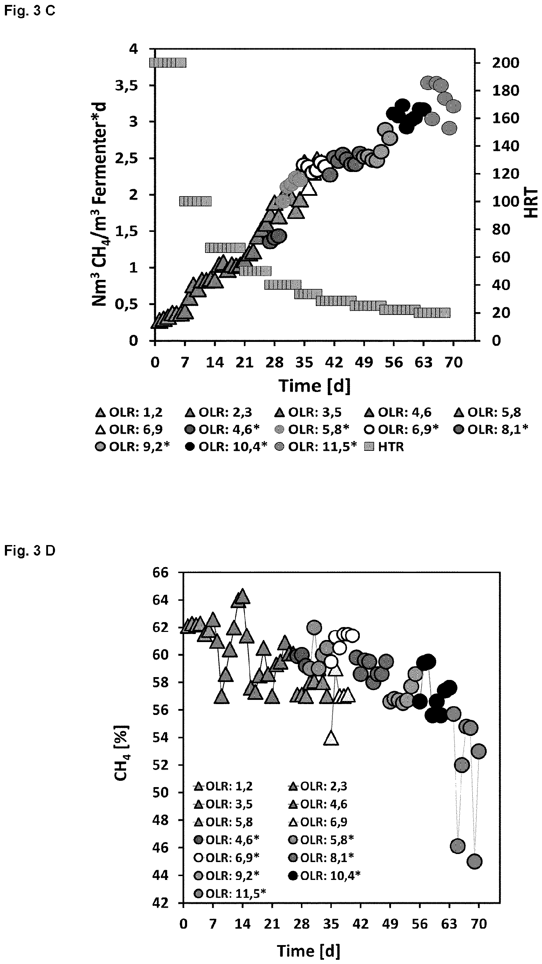

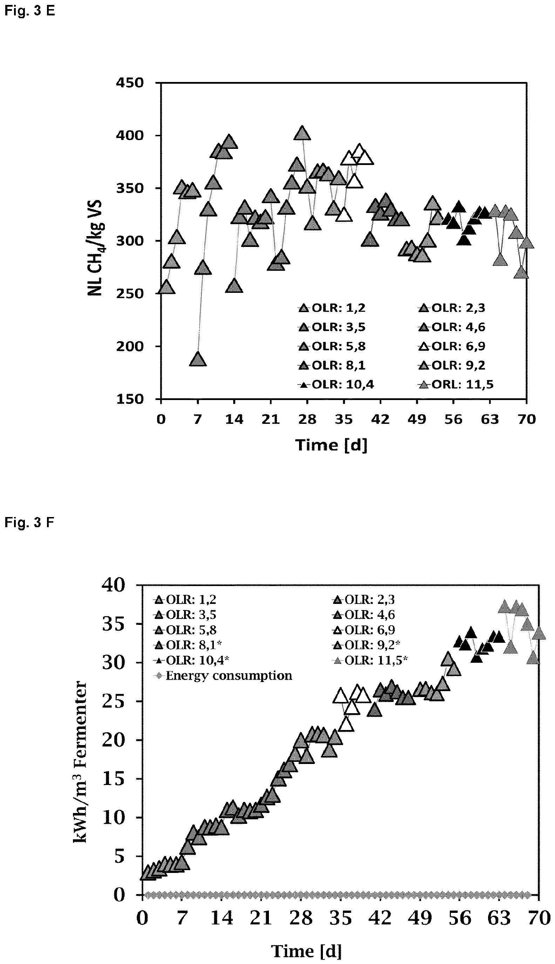

**Fig. 3** **A shows the accumulated methane production from the semi-continuous operation of sugar beet pulp (T:** 46 °C; HRT: **50 days (d)).**

Sugar beet pulp collected from the biogas plant of Anklam was operated with a hydraulic retention time (HRT) of 50 d, the organic loading rate (OLR) of is approx. 1 kg VS/m³*d. The fermenter was fed every 3,5 days (two times a week). Initially, the enzymatic pretreatment (E) was carried out by the mix of enzyme solution (enzyme powder + deionized water) with SBP and led into incubation for 48 h at 50 °C. After the incubation, the mixture was fed into the fermenter. The magnetized enzyme-substrate mixture (E-MF) was performed in the same way as the (E) pretreatment, but the mixture was exposed to a MF before the incubation step.

**Fig. 3** **B** shows the methane content present in biogas from the semi-continuous operation of sugar beet pulp. **Fig. 3** **C** shows the results in terms of the OLR (organic loading rate), wherein in **Fig. 3** **D** shows the methane content for different OLRs. **Fig. 3** **E** corresponds to the normalized methane production of organic mass. **Fig. 3** **F** shows the energy production from the pretreatment and energy consumption to perform the pretreatment with increasing OLR.

The 200 L fermenter was fed daily. The operation in this scale was done to validate the results from the 10 L fermenter. Initiating with a control (substrate without pretreatment), followed by an enzymatic pretreatment and then, with the enzymatic and magnetic pretreatment (E-MF). After operating the E-MF with an OLR of 4,5, this value was increase to 7 and successfully operated, indicating that it is possible to increase the OLR and have a stable operation.

This increase in OLR was tested due to previous results. The operation of the 10 L fermenter showed that the process was stable until the OLR of 6,9, reaching a stagnation after this value, due to foam formation, but increasing afterwards on the OLR of 10 and 11, with the addition of antifoam agent. As the operation in higher OLRs would also be problematic due to instability in the process, the OLR of 7 was chosen as it is more realistic for an industrial biogas plant.

**Fig. 4** **A** summarizes the results of the normalized biogas production in the 200 L fermenter for the control, enzymatic and E-MF pretreatment. The methane production of the E-MF pretreatment is clearly higher than the control and the enzyme pretreatment **(****Fig. 4 B)****.** The production per hour **(****Fig. 4 C)** shows that the methane production is not only higher and faster but also there are more carbon conversion to methane than the control and enzymatic pretreatment since the production on the end of the curve is higher than the enzymatic pretreatment and control. When investigating higher OLRs, the increase of methane production for the OLR of 7 was kept **(****Fig. 4** **D & 4 E).**

The methane percentage from the E-MF pretreatment is in average 5 % higher than the control and enzymatic pretreatment **(****Fig. 4 F)****.** With increasing OLR, this increase was slightly decreased but still higher than the control and enzymatic pretreatment (see **Fig. 4 G)****.** **Fig. 4** **G** shows the biomethane content measured in biogas for the enzymatic-magnetic pretreatment (E-MF) with a temperature of 44 °C, OLR: 4,5; 5,4; 6,2 & 7 (kgVS/m³d).

**Fig. 4** **H** shows the methane content of the whole experiment until this date, measured from the biogas for the control, enzymatic pretreatment and enzymatic-magnetic pretreatment (E-MF) at OLR: 4,5, and then at higher OLRs: 5,4; 6,2 & 7 (kgVS/m³d) at a temperature 44 °C.

The energy production increased with increasing OLR (see **Fig. 4 I)****,** which is indicating that operating at a higher OLR (7) will yield more energy production. This logic would not be true without the pretreatment application. In an industrial biogas plant, there is a limiting OLR the plant operates, going higher than this limit will decrease the energy production as the process will start to be inefficient. With the application of this pretreatment, it is showed that going to OLR 7 is feasible and promotes a higher energy production.

The energy consumption for the pretreatment in this scale (200 L) is negligible (0,0046 Wh), compared to the energy production (kWh). The energy consumed by the pretreatment in the 10L fermenterwas 0.08 Wh. The energy consumption increases with increasing cooper wire due to the resistivity of the cooper.

Also, the energy consumption does not depend on the amount of substrate that is treated (as usually happens with pretreatment in anaerobic digestion), meaning that the energy consumption to perform the pretreatment will not increase with increasing mass flow in the scale up. This was already seen in the 10 L fermenter and now validated with the 200 L fermenter.

**Fig. 4** **J** shows different methane yields, i.e., the methane production per mass, for different OLRs. This graphic shows that the efficiency in energy production will not decrease with increasing OLR, what would happen without the application of the pretreatment.

Another point is that is not necessary to treat 100 % of the daily feed to reach these results. As we kept the magnetization pipe length constant with increasing OLR, the pretreated mass decreased with increasing OLR (see **Fig. 4 K)****,** validating that is not necessary to pretreat 100 % of the mass.

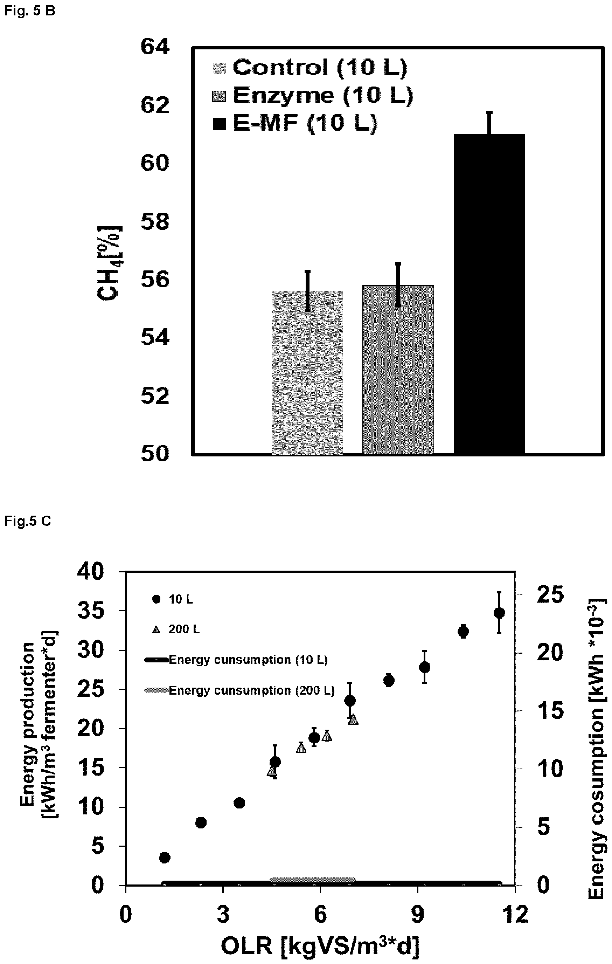

**Fig. 5** **A and B** serves to compare the methane production of the 10 and 200 L fermenter. These two graphics indicates that the results obtained in the 10 L fermenter (OLRs of 4,6; 5,8 & 6,9) is validated by the 200 L fermenter (OLRs: 4,5; 5,4; 6,2 & 7). Also, **Fig. 5** **C** shows that the energy production (kWh/m³ of fermenter) of both operations are also in the same range, validating the efficiency of the pretreatment application. On the secondary axis, is the energy consumption by the solenoid in Watts (kWh*10⁻³).

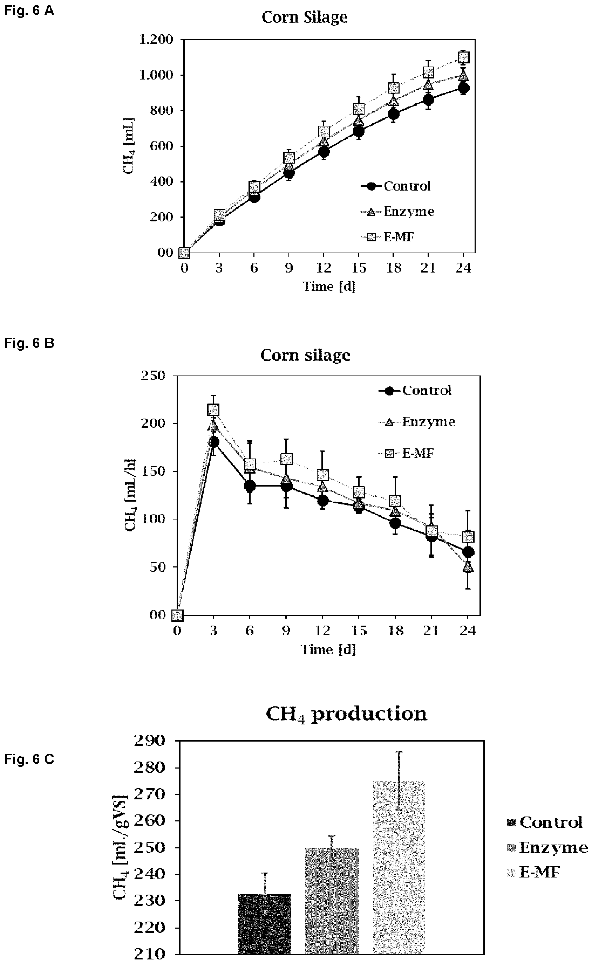

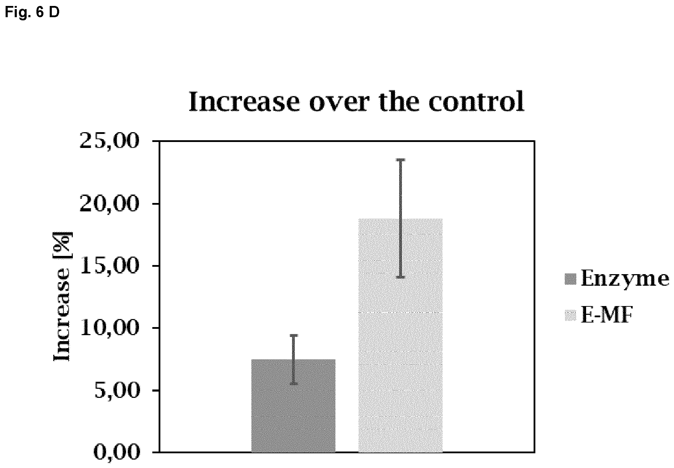

**Fig. 6** **A & B** Shows the cumulative methane production for the investigations on the process application for corn silage. In theses graphics is clear that the magnetization of the fermentation broth-substrate-enzyme mixture increases the methane production of this substrate.

**Fig. 6** **C** shows the total increase in methane production per gram of organic mass and **Fig. 6** **D** shows the percentage increase that the E-MF pretreatment achieves in comparison with the enzymatic pretreatment for corn silage.

### BIBLIOGRAPHY

Haritwal, Anurag, et al. "Study on the Improved Biogas Generation Through Magnetic Field Modified Anaerobic Digestion." International Journal Engineering Research & Technology 4.5 (2015): 1175-1179.
Zablodskiy, Mykola, Petr Klendiy, and Volodymyr Gritsyuk. "The Influence of a Rotating Magnetic Field on the Intensity of Methane Formation in a Bioreactor." 2019 IEEE 39th International Conference on Electronics and Nanotechnology (ELNANO). IEEE, 2019.
Litti, Yury, et al. "Increasing the efficiency of organic waste conversion into biogas by mechanical pretreatment in an electromagnetic mill." Journal of Physics: Conference Series. Vol. 1111. No. 1. IOP Publishing, 2018.
Zablodskiy, Mykola, et al. "Intensification of Biogas Fermentation Processes in the Bioenergy System." 2018 IEEE 3rd International Conference on Intelligent Energy and Power Systems (IEPS). IEEE, 2018.
B.E. Wood, H.C. Aldrich, L.O. Ingram. Ultrasound stimulates ethanol production during the simultaneous saccharification and fermentation of mixed waste office paper. Biotechnol Progr.13 (1997) 232-7.
Li CZ, Yoshimoto M, Ogata H, Tsukuda N, Fukunaga K, Nakao K. Effects of ultrasonic intensity and reactor scale on kinetics of enzymatic saccharifica- tion of various waste papers in continuously irradiated stirred tanks. Ultrason Sonochem;12 (2005) 373-84.
Bentsman et al. Modeling and indentification of the dynamics of the MF-influenced free-radical transformations in lipid-modeling substances and lipids. 2006.

## Claims

1. Method to produce biogas from organic substrates comprising:
a. Providing an organic substrate,
b. Providing an enzyme,
c. Recirculating a fermentation broth from a fermenter and mixing the fermentation broth with the organic substrate and the enzyme to form a fermentation broth-enzyme-substrate complex,
d. Exposing the fermentation broth-enzyme-substrate complex to a magnetic field for a time t₁,
e. Introducing magnetically treated fermentation broth-enzyme-substrate complex into the fermenter.

2. Method according to claim 1
**characterized in that**
at least a fraction of the fermentation broth or a whole fermentation broth from the fermenter is recirculated and mixed with the with the organic substrate and the enzyme to form the fermentation broth-enzyme-substrate complex.

3. Method according to one or more of the preceding claims
**characterized in that**
the fermentation broth is mixed with the organic substrate and the enzyme in a temperature range between 30 - 70°C, preferred between 35 - 60°C, particularly preferred between 44 - 56°C, very particularly preferred between 40 - 55°C.

4. Method according to one or more of the preceding claims
**characterized in that**
the time t₁ is selected from a range between 1 - 6 h, preferred between 3 - 5 h, particularly preferred 4 h.

5. Method according to one or more of the preceding claims
**characterized in that**
while forming the fermentation broth-enzyme-substrate complex the fermentation broth-enzyme-substrate is surrounded by a static magnet field.

6. Method according to one or more of the preceding claims
**characterized in that**
recirculating the fermentation broth from the fermenter is performed by a pump, preferably a positive displacement pump.

7. Method according to one or more of the preceding claims
**characterized in that**
the magnetic field has a magnetic flux density between 0.01 - 10 mT, preferably 0.2 mT.

8. Method according to one or more of the preceding claims
**characterized in that**
the magnetic field is provided by a solenoid through which a DC current flows.

9. Method according to one or more of the preceding claims
**characterized in that**
the organic substrate is selected from a group comprising sugar beet pulp, corn silage, straw and/or gluten, which is preferably contained in the fermentation broth..

10. Method according to one or more of the preceding claims
**characterized in that**
the enzyme is selected from a group comprising pectinase, cellulase, xylanase and/or glucanase.
